# EUROPEAN PATENT APPLICATION

(11) **EP 3 305 989 A1**
(43) Date of publication of application: **11.04.2018**
(21) Application number: 17194279.0
(22) Date of filing: 02.10.2017
(51) Int. Cl.: E02D 1/02

(54) **PENETROMETER**

(30) Priority: 04.10.2016 NL 2017574
(71) Applicant: Fugro Engineers B.V., 2631 RT Nootdorp (NL)
(72) Inventor: LOOIJEN, Peter Nicolaas, 2631 RT Nootdorp (NL); RUIJTENBEEK, David Steven, 2631 RT Nootdorp (NL)
(74) Representative: Van Breda, Jacobus

(57) **Abstract**

Penetrometer (1) arranged for detecting a transition zone from water to sediment of a seabed, comprising a cone (2) with a cone sleeve (3) and a cone tip (4) and a force sensitive element or elements (5, 10), wherein the force sensitive element or elements (5, 10) comprise a piezo-electric sensor (5) which is provided in contact with the cone tip (4) so as to respond to a force or forces applied to the cone tip (4). The piezo-electric sensor (5) is supported by a rim (6) inside the cone sleeve (3).

## Description

The invention relates to a penetrometer arranged for detecting a transition zone from water to sediment of a seabed, comprising a cone with a cone sleeve and a cone tip and a force sensitive element or elements.

Such a penetrometer is used in practice for detecting the exact position of the transition from water to the sediment of the seabed. This level forms a reference point for a subsequent soil measurement below the sea bottom, wherein it is a necessity to exactly know the level of penetration of the penetrometer with reference to the level of the seabed.

The known penetrometer used by industry is equipped with sensors embodied as strain gauges that are used for measuring a force reaction of the soil. In practice however this measurement with the known penetrometer equipped with strain gauges does not suffice because the top layer of the seabed is too soft to accurately measure the level of the seabed.

It is an object of the invention to provide a solution for this problem.

From GB-A-2466773 a penetrometer is known, comprising a cone with a cone sleeve and a cone tip and a force sensitive element or elements that may comprise piezo-electric elements, which is or are provided in contact with the cone tip so as to respond to a force or forces applied to the cone tip.

The penetrometer of the invention has the features of one or more of the appended claims.

In a first aspect of the invention the piezo-electric element is a piezo-electric sensor that is supported by a rim inside the cone sleeve. The force applied to the piezo-electric sensor is converted in an electrical signal which indicates when the penetrometer of the invention has reached the level of the seabed. Supported by the rim the piezo-electric sensor can thus be subjected to the force of the cone tip without being moved out of position to provide that the measurement of the seabed level is reliable and accurate. Accordingly the penetrometer can be effectively used for detecting a transition from water to sediment of a seabed.

Beneficially the cone tip is provided with a shoulder arranged to cooperate with a frontal end of the cone sleeve for limiting a movement range of the cone tip in a direction towards the piezo-electric sensor. In this way damage to the piezo-electric sensor due to excessive loads by the cone tip is prevented.

Advantageously the cone sleeve is comprised within an outer sleeve, wherein the cone sleeve and the outer sleeve are longitudinally movable with respect to each other. By this arrangement it is possible that between the cone sleeve and the outer sleeve one or more high load force sensors, preferably strain gauges, are applied. The high load force sensors then can provide measurement signals of the force applied to the cone tip in a force measurement range for which the piezo-electric sensor is not suitable and would be prone to damage. The high load force sensors, preferably strain gauges, can in particular be used for force measurement when the shoulder of the cone tip rests on the frontal end of the cone sleeve.

The invention will hereinafter be further elucidated with reference to the drawing of an exemplary embodiment of an apparatus according to the invention that is not limiting as to the appended claims.

In the drawing:
- figure 1 shows a first embodiment of the penetrometer according to the invention; and
- figure 2 shows a second embodiment of the penetrometer according to the invention.

Whenever in the figures the same reference numerals are applied, these numerals refer to the same parts.

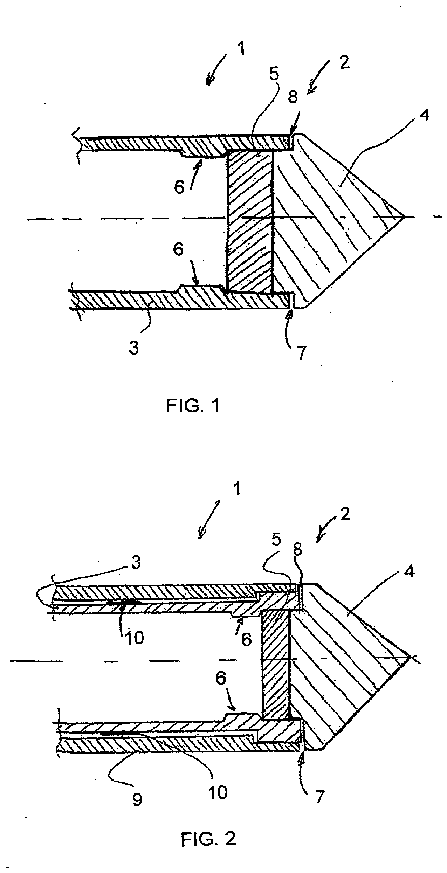

With reference to both figure 1 and figure 2, a first embodiment and a second embodiment of the penetrometer 1 of the invention is shown.

Both embodiments of figure 1 and figure 2 are arranged for detecting a seabed force and comprise a cone 2 with a cone sleeve 3 and a cone tip 4 and a force sensitive element or elements. The force sensitive element or elements comprise according to the invention at least a piezo-electric sensor 5 which is provided in contact with the cone tip 4 so as to respond to a force or forces applied to the cone tip 4.

The piezo-electric sensor 5 is in both embodiments of figure 1 and figure 2 supported by a rim 6 inside the cone sleeve 3.

Further the cone tip 4 is in both embodiments of figure 1 and figure 2 provided with a shoulder 7 arranged to cooperate with a frontal end 8 of the cone sleeve 3 for limiting a movement range of the cone tip 4 in a direction towards the piezo-electric sensor 5.

The embodiment shown in figure 2 differentiates from the embodiment shown in figure 1 in that the cone sleeve 3 is comprised within an outer sleeve 9, wherein the cone sleeve 3 and the outer sleeve 9 are longitudinally movable with respect to each other.

This makes possible that between the cone sleeve 3 and the outer sleeve 9 one or more strain gauges 10 or other high load force sensors are applied.

Although the invention has been discussed in the foregoing with reference to exemplary embodiments of the penetrometer of the invention, the invention is not restricted to these particular embodiments which can be varied in many ways without departing from the invention. The discussed exemplary embodiments shall therefore not be used to construe the appended claims strictly in accordance therewith. On the contrary the embodiments are merely intended to explain the wording of the appended claims without intent to limit the claims to these exemplary embodiments. The scope of protection of the invention shall therefore be construed in accordance with the appended claims only, wherein a possible ambiguity in the wording of the claims shall be resolved using these exemplary embodiments.

## Claims

1. Penetrometer (1) arranged for detecting a transition from water to sediment of a seabed, comprising a cone (2) with a cone sleeve (3) and a cone tip (4) and a force sensitive element or elements (5, 10), wherein the force sensitive element or elements (5, 10) comprise a piezo-electric element (5) which is provided in contact with the cone tip (4) so as to respond to a force or forces applied to the cone tip (4), **characterized in that** the piezo-electric element is a piezo-electric sensor (5) that is supported by a rim (6) inside the cone sleeve (3).

2. Penetrometer according to claim 1, **characterized in that** the cone tip (4) is provided with a shoulder (7) arranged to cooperate with a frontal end (8) of the cone sleeve (3) for limiting a movement range of the cone tip (4) in a direction towards the piezo-electric sensor (5).

3. Penetrometer according to claim 1 or 2, **characterized in that** the cone sleeve (3) is comprised within an outer sleeve (9), wherein the cone sleeve (3) and the outer sleeve (9) are longitudinally movable with respect to each other.

4. Penetrometer according to claim 3, **characterized in that** between the cone sleeve (3) and the outer sleeve (9) are one or more high load force sensors, preferably strain gauges (10).

5. Method of using a penetrometer (1) for detecting a sediment layer, wherein the penetrometer comprises a cone (2) with a cone sleeve (3) and a cone tip (4) and a force sensitive element or elements (5, 10),wherein the force sensitive element or elements (5, 10) comprise a piezo-electric sensor (5) which is provided in contact with the cone tip (4) so as to respond to a force or forces applied to the cone tip (4), **characterized in that** the penetrometer is used for detecting a transition from water to sediment of a seabed.
